(19) **Europäisches Patentamt**

**European Patent Office**

**Office européen des brevets**

(11) Numéro de publication: **0 401 170**

**A1**

(12) ## DEMANDE DE BREVET EUROPEEN

(21) Numéro de dépôt: **90810385.6**

(51) Int. Cl.⁵: **A61F 5/01**

(22) Date de dépôt: **25.05.90**

(30) Priorité: **30.05.89 SU 202389**

(43) Date de publication de la demande:
**05.12.90 Bulletin 90/49**

(84) Etats contractants désignés:
**DE FR GB IT**

(71) Demandeur: **Vanden Broeck, Marc**
**14b, route du Curson**
**CH-1197 Prangins(CH)**

Demandeur: **De Marchi, Olivier**
**Ecole de Duillier .**

**CH-1266 Duillier(CH)**

(72) Inventeur: **Vanden Broeck, Marc**
**14b, route du Curson**
**CH-1197 Prangins(CH)**
Inventeur: **De Marchi, Olivier**
**Ecole de Duillier**
**CH-1266 Duillier(CH)**

(74) Mandataire: **Dousse, Blasco et al**
**7, route de Drize**
**CH-1227 Carouge/Genève(CH)**

(54) **Dispositif d'articulation de deux armatures d'orthèse.**

(57) Chaque armature d'orthèse (17, 18) comporte un secteur denté (15, 16) pivotant autour d'un axe. Ces secteurs dentés sont en prise l'un avec l'autre et sont montés entre deux flasques parallèles (1) séparées par deux entretoises (3, 4). Des ouvertures (21, 22) traversant ces flasques sont réparties angulairement autour de chaque axe de pivotement et sont destinées à recevoir sélectivement des butées formées par des chevilles (25, 26, 27, 28) et délimitant les secteurs de liberté des armatures (17, 18) autour des axes de pivotement. Ces butées sont associées chacune à une languette percée qui s'étend perpendiculairement à l'axe de la cheville et permet de verrouiller la butée au centre de pivotement de son armature (17, 18).

FIG. 1

## DISPOSITIF D'ARTICULATION DE DEUX ARMATURES D'ORTHESE

La présente invention se rapporte à un dispositif d'articulation de deux armatures d'orthèse destinées à être associées aux organes de fixation respectifs de l'orthèse au corps humain, comprenant deux flasques de support solidaires et parallèles l'une à l'autre, deux tourillons disposés entre ces flasques, deux secteurs dentés semi-circulaires montés coaxialement sur les tourillons respectifs, en prise l'un avec l'autre et solidaires des armatures respectives et des moyens de butées réglables s'étendant dans la trajectoire d'au moins une de ces armatures pour définir son secteur de liberté.

Les orthèses du genou comportent des moyens de fixation au-dessus et au-dessous du genou reliés à deux dispositifs d'articulation situés sur les parties interne et externe du genou.

Comme on le sait, le mouvement de flexion-extension de l'articulation du genou se produit par roulement et glissement des condyles fémoraux sur les ménisques du plateau tibial et non autour d'un centre de rotation fixe. C'est la raison pour laquelle les armatures reliant les organes de fixation de l'orthèse au corps humain ne sont généralement pas articulées autour d'un axe commun, mais autour de deux axes parallèles. Les deux armatures associées au même dispositif d'articulation sont reliées cinématiquement, par exemple par deux secteurs dentés en prise l'un avec l'autre. Il est également connu de définir un secteur de liberté de ces armatures en disposant des butées dans leurs trajectoires en fonction du mouvement de flexion-extension auquel on veut limiter l'articulation à laquelle l'orthèse est fixée. Ce choix est déterminé par le médecin qui doit lui-même effectuer sur l'orthèse le réglage des butées dans les trajectoires des armatures du dispositif d'articulation de l'orthèse.

Il existe à cet effet différents systèmes de réglage qui présentent l'inconvénient de nécessiter des outils tels que tournevis en particulier et des opérations de démontage et de montage auxquelles le médecin est moins familier que l'orthopédiste.

On a proposé notamment dans le US-A-4,817,588 un tel système de réglage dans lequel des éléments de butée sont positionnés dans des encoches réparties angulairement autour de l'axe d'articulation d'une orthèse, un organe de verrouillage servant à retenir ces éléments de butée dans leurs encoches respectives. L'inconvénient de ce système de réglage réside essentiellement dans le fait qu'il est relativement complexe et nécessite de ce fait la fabrication d'un assez grand nombre de pièces conduisant à des opérations de montage dont la durée et la difficulté sont fonction du nombre de pièces à assembler.

Le EP-A-0 059 472 ainsi que le US-A-4,726,361 décrivent un système de réglage plus simple comprenant des butées disposées le long de rainures en arc de cercle centrées sur les axes d'articulation et positonnées le long de ces rainures par serrage à l'aide d'une vis et d'un écrou disposés de part et d'autre du plan de la flasque dans laquelle est ménagée la rainure. L'inconvénient d'un tel système est que la position angulaire n'est définie que par le serrage des butées. Compte tenu de la force qui est appelée à s'exercer sur ces butées en raison de la longueur du bras de levier, la position de ces butées pourrait être accidentellement modifiée.

Une autre solution proposée dans le US-A-4,493,316 comporte des pignons portés par des bras serrés sur les axes d'articulation des deux branches de l'orthèse et en prise chacun avec une denture solidaire de chacun de ces axes, de sorte que ces pignons se déplacent angulairement avec ces dentures et limitent le déplacement angulaire lorsqu'ils rencontrent l'autre denture solidaire de l'axe d'articulation de l'autre branche de l'orthèse. Cette solution ne permet pas de déterminer des positions fixes celles-ci étant toujours fonction d'un serrage et donc susceptibles de se déplacer accidentellement.

Le même inconvénient se retrouve dans le GB-A-2 182 714, les déplacements angulaires des branches de l'orthèse étant limités par des vis de butée susceptibles de se dérégler.

Comme on peut le constater, il n'existe pas dans l'état de la technique de dispositif de réglage simple susceptible de définir des angles d'articulation inéréglables, c'est-à-dire des positions de butée qui ne sont pas tributaires d'une simple force de serrage. Par ailleurs, tous ces dispositifs nécessitent un outillage particulier non habituel chez un médecin.

Le but de la présente invention est de faire en sorte que cette opération de réglage du secteur de liberté des armatures ne nécessite notamment aucun outil spécial, ni démontage du dispositif d'articulation.

A cet effet, la présente invention a pour objet un dispositif d'articulation de deux armatures d'orthèse selon la revendication 1.

Le dessin annexé illustre, schématiquement et à titre d'exemple, une forme d'exécution du dispositif d'articulation objet de la présente invention.

La figure 1 est une vue en plan de ce dispositif.

La figure 2 est une vue en coupe développée à plus grande échelle selon la ligne II-II de la figure 1.

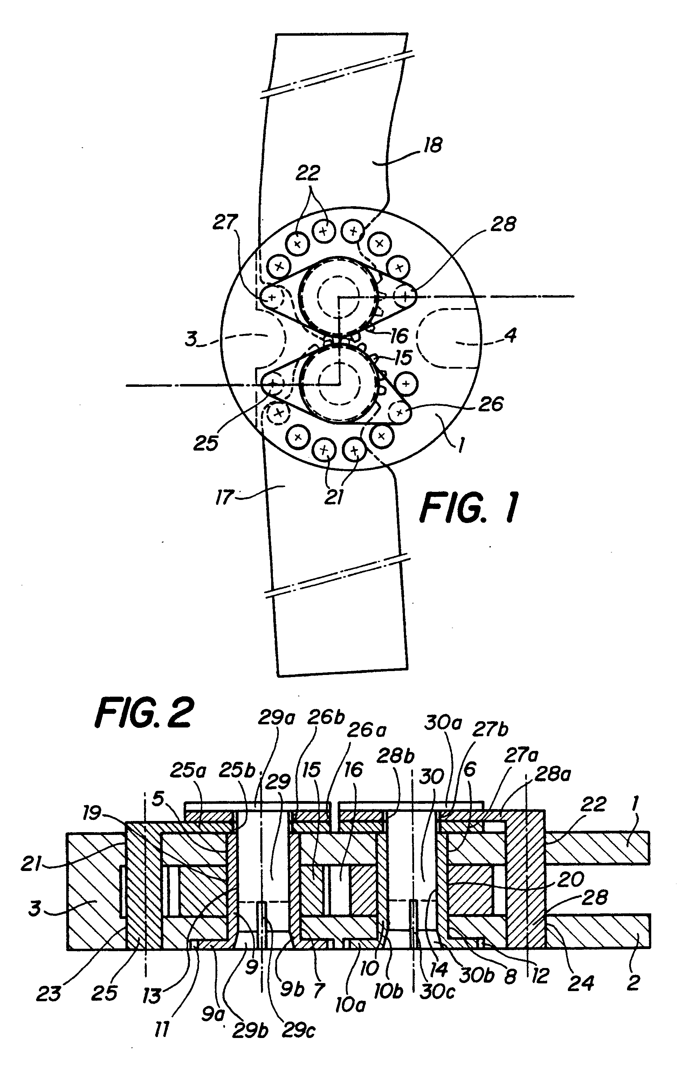

Le dispositif d'articulation illustré par les figures 1 et 2 comporte deux flasques de support parallèles 1, 2 maintenues écartées, l'une de l'autre par des entretoises 3, 4. Ces flasques et ces entretoises peuvent avantageusement venir d'une seule pièce par moulage en une matière plastique telle que le polyéthylène par exemple. Ces flasques comportent chacune deux ouvertures 5, 6 respectivement 7, 8 alignées sur deux axes respectifs formant deux centres d'articulations comme on le décrira par la suite.

Deux tourillons tubulaires 9, 10 sont ajustés dans les deux ouvertures alignées 5, 7 respectivement 6, 8 des deux centres d'articulation. Chaque tourillon comporte, à une extrémité, une collerette annulaire, 9a respectivement 10a. Ces collerettes prennent appui dans des dégagements 11 respectivement 12 ménagés dans la face externe de la flasque 2 autour des ouvertures 7 et 8. Les extrémités des passages axiaux 13, 14 des tourillons tubulaires 9, 10, adjacentes aux collerettes annulaires 9a, 10a, présentent des évasements coniques 9b, 10b.

Deux secteurs dentés 15, 16 solidaires respectivement de deux armatures 17, 18, destinées à être associées aux organes de fixation respectifs de l'orthèse au corps humain, comportent chacun une ouverture axiale 19, 20 servant à monter les armatures 17, 18, pivotantes sur les tourillons tubulaires 9, 10 en maintenant les secteurs dentés 15, 16 en prise l'un avec l'autre, de sorte que les deux armatures 17, 18 sont cinématiquement solidaires l'une de l'autre.

Chacune des ouvertures 5, 6, 7, 8 alignées deux à deux sur les axes respectifs des tourillons 9, 10 est entourée d'ouvertures de positionnement respectivement 21,22,23,24, réparties angulairement sur des circonférences de mêmes rayons et traversant les flasques respectives 1, 2. Les ouvertures 21 respectivement 22 coïncident avec les ouvertures 23, respectivement 24, c'est-à-dire qu'elles ont deux à deux des axes respectifs communs parallèles aux axes d'articulations passant par les centres des tourillons 9, respectivement 10.

Les moyens de butées réglables sont formés par des tiges 25, 26, respectivement 27, 28 qui peuvent être introduites dans n'importe quelle paire d'ouvertures de positionnement 21, 23 respectivement 22,24.

Chaque tige 25, 26, 27, 28 est solidaire à une extrémité, d'une plaquette 25a, 26a, 27a, respectivement 28a qui s'étend perpendiculairement à l'axe longitudinal de la tige. Chaque plaquette est percée d'une ouverture 25b, 26b, 27b, 28b dont la distance entre son centre et l'axe longitudinal de la tige correspond au rayon de cercle sur lequel sont réparties les ouvertures 21, 22, 23, 24 autour ues axes longitudinaux des tourillons 9, 10. Par conséquent, ces ouvertures peuvent être amenées à coïncider avec les passages axiaux 13, 14 des tourillons tubulaires respectifs 9, 10, lorsque les tiges sont engagées dans les ouvertures de positionnement.

Deux organes de fixation sont constitués chacun par une tige 29 respectivement 30. Ces tiges sont engagées dans les passages axiaux 13 respectivement 14, des tourillons tubulaires 9 respectivement 10, ainsi que dans les ouvertures 25b, 26b respectivement 27b, 28b, des plaquettes 25a, 26a, 27a, 28a. Chaque tige 29, 30 présente, à une extrémité, une tête 29a, 30a qui prend appui contre la plaquette externe 25a respectivement 27a et, à son autre extrémité, une partie évasée 29b, 30b dans laquelle deux fentes diamétrales 29c, 30C ménagent quatre bras élastiques entre-elles. Grâce à cette disposition, les parties évasées 29b, 30b des tiges 29 respectivement 30 peuvent se crocher dans les évasements coniques 9b respectivement 10b après avoir traversé les passages axiaux 13, 14 des tourillons tubulaires 9, 10.

Pour monter les armatures 17, 18 sur le dispositif d'articulation, il suffit d'introduire latéralement leurs secteurs dentés 15 et 16 entre les flasques 1 et 2 et de faire coïncider leurs ouvertures axiales 19, 20 avec les ouvertures 5, 7 respectivement 6, 8 des flasques. Ensuite, on ajuste chacun des tourillons tubulaires 9, 10 à travers ces ouvertures 5, 7, 19 respectivement 6, 8, 20. L'articulation est, dès ce moment, en état de fonctionnement, mais aucun réglage particulier du secteur dans lequel chaque armature 17, 18 est libre de se déplacer n'est fixé, si ce n'est les limites maximum inhérentes à la construction du dispositif.

Si on désire limiter, voire empêcher le mouvement de flexion-extension du genou à laquelle l'orthèse doit être fixée, on introduit les tiges 25, 26, respectivement 27, 28 dans les ouvertures de positionnement 21, 23 respectivement 22, 24 en fonction des angles d'extension et de flexion désirés pour cette articulation. Etant donné que les deux armatures 17, 18 sont reliées cinématiquement l'une à l'autre, théoriquement seul un axe d'articulation de l'un des tourillons 9, 10 pourrait être muni de moyens de butées réglables. Toutefois, compte tenu des efforts importants qui sont appelés à solliciter le dispositif, il est nécessaire que chaque armature soit associée à deux butées et que ces butées soient réglées pour que les deux armatures arrivent simultanément contre les deux butées respectives définissant chacune des deux extrémités de leurs déplacements angulaires. Une fois que le réglage désiré a été obtenu, il suffit d'introduire axialement les tiges 29, 30 des organes de fixation dans les passages axiaux 13 respectivement 14 des tourillons tubulaires 9, 10 en les poussant jusqu'à ce que leurs parties évasées respectives 29b, 30b se crochent dans les évasements 9b, 10b de ces passages axiaux.

Pour l'enlèvement de ces organes de fixation, il suffit d'appliquer une pression axiale contre l'extrémité de la partie évasée 29b ou 30b. Compte tenu du fait que les tourillons 9, 10 sont retenus dans cette direction par leurs collerettes annulaires 9a, 10a et que les évasements 9b, 10b sont coniques, les bras élastiques formés par les fentes 29c, 30c se contractent radialement vers le centre et permettent le coulissement des tiges 29, 30 hors des passages axiaux 13, 14. Pour effectuer ces opérations, aucun outil particulier n'est nécessaire, une pointe quelconque de crayon, de ciseaux par exemple est suffisante pour extraire les organes de fixation des passages axiaux des tourillons. Pendant que l'on exerce cette poussée axiale sur les organes de fixation, les tourillons 9, 10 sont retenus par les collerettes 9a, 10a. Les tourillons eux ne peuvent être dégagés qu'en exerçant une poussée axiale à leurs extrémités respectives opposées. Toutefois, cette opération n'est pas nécessaire lors du réglage du degré de liberté du dispositif d'articulation. Il faut cependant relever que, grâce à cette disposition selon laquelle les organes de fixation et les tourillons peuvent être extraits axialement par des poussées axiales exercées selon des directions respectives opposées, le support d'articulation formé par les flasques 1, 2 et les entretoises 3, 4 peut être réalisé en une seule pièce moulée puisque les flasques n'ont pas à être séparées pour le montage des armatures sur les tourillons. Cette disposition réduit le nombre de pièces et facilite le montage de l'articulation qui ne nécessite aucun outil lui non plus.

## Revendications

1. Dispositif d'articulation de deux armatures d'orthèse destinées à être associées aux organes de fixation respectifs de l'orthèse au corps humain comprenant deux flasques de support solidaires et parallèles l'une à l'autre, deux tourillons disposés entre ces flasques, deux secteurs dentés semi-circulaires montés coaxialement sur les tourillons respectifs, en prise l'un avec l'autre et solidaires des armatures respectives et des moyens de butées réglables s'étendant dans la trajectoire d'au moins une de ces armatures pour définir son secteur de liberté, caractérisé par le fait qu'au moins l'une desdites flasques comporte des ouvertures de positionnement réparties angulairement autour de l'axe d'au moins l'un des tourillons, deux butées d'écartement angulaire réglables par rapport à l'axe de ce tourillon, chacune de ces butées comportant une tige s'étendant à travers une ouverture de positionnement de ladite flasque, cette tige étant solidaire d'une plaquette, dont le plan est perpendiculaire à l'axe longitudinal de cette tige, comportant des moyens de liaison à l'axe dudit tourillon.

2. Dispositif d'articulation selon la revendication 1, caractérisé par le fait que le tourillon est traversé par une ouverture axiale cylindrique dont une extrémité comporte un évasement, que chaque plaquette est munie d'une ouverture destinée à coïncider avec l'autre extrémité de ladite ouverture axiale et qu'un organe de fixation passe à travers ces ouvertures et comporte une tige munie à une extrémité d'une tête prenant appui contre l'une desdites plaquettes et, à son autre extrémité, d'une partie évasée correspondant à celle de l'ouverture axiale, deux fentes diamétrales ménageant quatre éléments élastiques radialement dans cette partie évasée dudit organe de fixation.

3. Dispositif d'articulation selon la revendication 2, caractérisé par le fait que ledit tourillon est formé d'une pièce rapportée dans deux ouvertures coaxiales desdites flasques et comprend une partie tubulaire dont l'extrémité comportant ledit évasement est entourée d'une collerette annulaire de retenue axiale.

4. Dispositif selon la revendication 1, caractérisé par le fait que lesdits moyens de butées réglables s'étendent dans la trajectoire de chacune desdites armatures.

FIG. 1

FIG. 2

**Office européen des brevets**

# RAPPORT DE RECHERCHE EUROPEENNE

Numero de la demande

EP 90 81 0385

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,Y | EP-A-0 059 472 (UNITED STATES MANUFACTURING)<br>* Document en entier * | 1 | A 61 F 5/01 |
| A | | 2-4 | |
| D,Y | US-A-4 493 316 (K.E. REED et al.)<br>* Abrégé; Figures 4,6 * | 1 | |
| A | | 2-3 | |
| D,A | GB-A-2 182 714 (D.E. YOUNG et al.)<br>* Page 1, lignes 112-130; page 2, lignes 66-72,90-109,123-126; figure 5 * | 4 | |
| D,A | US-A-4 726 361 (M.D. FARLEY)<br>* Colonne 4, lignes 30-37; figure 3 * | 1,4 | |
| D,A | US-A-4 817 588 (G.R. BLEDSOE)<br>* Colonne 1, lignes 5-43; colonne 14, ligne 64 - colonne 20, ligne 2; figures 6-13 * | 1 | |

DOMAINES TECHNIQUES RECHERCHES (Int. Cl.5)

A 61 F

Le présent rapport a été établi pour toutes les revendications

| Lieu de la recherche | Date d'achèvement de la recherche | Examinateur |
|---|---|---|
| LA HAYE | 29-08-1990 | NICE P.R. |